# EUROPEAN PATENT SPECIFICATION

(11) **EP 1 615 564 B1**
(45) Date of publication and mention of the grant of the patent: **23.07.2014**
(21) Application number: 03778559.9
(22) Date of filing: 03.12.2003
(51) Int. Cl.: A61B 8/08, A61N 5/10

(54) **RESPIRATION MONITORING PROCESS AND DEVICE**
VERFAHREN UND GERÄT ZUR ATMUNGSÜBERWACHUNG
PROCEDE ET DISPOSITIF DE SURVEILLANCE RESPIRATOIRE

(30) Priority: 03.12.2002 GB 0228189
(43) Date of publication of application: 18.01.2006
(73) Proprietor: Neorad A/S, N-0256 Oslo (NO)
(72) Inventor: BRABRAND, Knut, N-1476 Rasta (NO)
(74) Representative: Taylor, Adam David
(86) International application number: PCT/GB2003/005211
(87) International publication number: WO 2004/049951

(56) References cited:
- EP-A- 0 940 158
- EP-A- 1 086 652
- EP-A- 1 208 796
- WO-A-02/41776
- US-A- 4 431 007
- KOKUBO M ET AL: "Non-invasive respiratory-gated radiation treatment system based on a 3-D ultrasound device and a 3-D digital localizer" INTERNATIONAL JOURNAL OF RADIATION ONCOLOGY BIOLOGY PHYSICS, vol. 54, no. 2 Supplement, 2002, page 314 XP002273267 44th Annual Meeting of the American Society for Therapeutic Radiology and Oncology;New Orleans, LA, USA; October 06-10, 2002 ISSN: 0360-3016
- ETLIK O. ET AL: "Demonstrating the effect of theophylline treatment on diaphragmatic movement in chronic obstructive pulmonary disease patients by MR-fluoroscopy", EUROPEAN JOURNAL OF RADIOLOGY, vol. 51, no. 2, August 2004 (2004-08), pages 150-154,

## Description

The present invention relates to a non-invasive method and apparatus for monitoring the degree of inspiration of a medical or surgical patient's lungs.

In many circumstances (e.g. when performing biopsies or radiotherapy), it is desirable to precisely locate a target area of a lung or of an organ in the upper abdomen. For example, in order to perform a lung biopsy, pre-operative CT images may be generated. These are then used to determine how the biopsy needle should be inserted in order to reach its target. After the needle has been inserted, fresh CT images may be generated in order to check the position of the needle. If necessary, this can then be corrected and the procedure repeated in an iterative manner.

During such a CT guided puncture, several parameters are of great importance in order to hit the target, e.g. insertion of the needle with the correct angle needle deflection at various tissue interfaces and patient cooperation, including respiratory control. Lesions in the lungs, particularly in the lower parts, and also in the upper abdominal area move with respiration. The degree of respiratory movement is greater in the lower parts of the lung and the upper part of the abdomen. Other organs and tissues move to some extent with respiration.

Although some x-ray based imaging techniques can be used in real-time, it is not desirable to continuously monitor a biopsy using x-rays because of the well known risks associated with exposure to such radiation. Magnetic implements cannot be used during MRI scans because of the high magnetic field used.

Conventionally, the patient is asked to hold his breath when the CT scan takes place. He is then asked to hold his breath at the same level of inspiration during puncture. However, it will be appreciated that it is difficult for the patient to reproduce the same degree of inspiration. Consequently, the images may not accurately reflect the position of the target within the patient. Thus, an objective means of monitoring the level of inspiration could improve the puncture accuracy because the operator would have better control over the movement of the target along the longitudinal body axis (the z-direction).

US 6,110,112 discloses a medical guide apparatus for breath-coordinated puncturing of the body. In this apparatus, a reference point is located which will be visible in both an ultrasound scan and in an MRI or CT scan. The arrangement is such that the location of the reference point will vary with respiration in the same manner as the target point. The target (which shows up in only the MRI or CT scan) can then be located by calculating its position relative to the reference point. The practitioner must calculate the relative displacements in the x, y and z-axes of the target point from the reference point.

The apparatus of US 6,110,112 consists of an ultrasound applicator with a puncture instrument attached thereto. The relative displacement between the ultrasound applicator and the puncture instrument is variable. The practitioner therefore sets the relative displacement of the puncture instrument from the ultrasound applicator such that the puncture instrument is directed at the target. Then, the practitioner locates the reference point with the ultrasound applicator and performs the puncture. This method requires that the reference point used moves in exactly the same way as the target area throughout the respiration cycle. It also requires a cumbersome apparatus to be mounted above the patient after the CT or MRI scan has been performed.

There is a similar problem of z-direction movement associated with radiation therapy of a tumor. If the tumor is in the lung or upper part of the abdomen, it may move in and out of the focus of the radiation beam as the lungs expand and contract. The current method of overcoming this problem is to choose the area to be irradiated to be large enough to cover the tumor during its respiratory movement. This inevitably results in the irradiation of non-cancerous tissue. The inventors have therefore recognized that a device that can monitor and send information about the respiration to a radiation unit would be desirable. This could enable a radiation unit to map the radiation beam as a function of the patient respiration and thus trigger the radiation unit to only radiate when the tumor is in focus.

WO 02/041776 discloses a system using ultrasound to track the motion of an organ, such as a diaphragm.

According to a first aspect of the invention there is provided a method of determining the degree of lung inspiration in a patient comprising the step of non-invasively detecting the position of the patient's diaphragm by means of an array of at least two ultrasound transducer elements on the patient extending in the direction of the longitudinal (z) axis of the patient over the lung sinus, wherein: each individual transducer element detects an ultrasound beam which is reflected from tissue adjacent thereto and provides an output signal;
and comprising the step of measuring the strength of each output signal to obtain a value for the acoustic impedance of said tissue adjacent to the transducer element; and determining the position of the diaphragm based upon a comparison of the measured acoustic impedance values from each transducer.

Thus, the invention is based on the recognition that the diaphragm provides an effective and measurable reference point that may be used to define lung inspiration.

The invention may thus be used to assist in image-guided procedures as discussed above. The diaphragm position may therefore be used to define the degree of lung inspiration when an image of the patient is generated and the method may further comprise the step of reproducing that degree of lung inspiration in order to perform a medical or surgical procedure on the patient based on that image.

For example, diaphragm position may first be determined and then, whilst the patient holds his breath, images may be generated. Subsequently, the degree of lung inspiration may be substantially reproduced by the patient inhaling until the previously determined diaphragm position is achieved. The desired procedure is then carried out whilst the patient holds his breath.

Ultrasound does not subject the patient to ionizing radiation and is convenient to use. Although ultrasound cannot image certain body structures it provides a very effective way to determine the position of the diaphragm.

A conventional hand-held ultrasound probe could be used to scan the patient's abdomen. However, this would still require a reference point to define the diaphragm's position and it would be difficult to monitor movement of the diaphragm.

The invention makes use of the fact that air has a very high acoustic impedance compared to non-aerated tissue (tissue not containing air). During inspiration, as the lung expands downwards and fills with air, the lung sinus opens up. The acoustic impedance of the lung sinus is thus increased. The opposite takes place during expiration. As a consequence, transducer elements located adjacent to aerated tissue will provide very distinct outputs compared to those adjacent to non-aerated tissue. In this way the invention allows accurate real time monitoring of the position of the diaphragm.

As the transducer elements are adapted to extend over the lung sinus, they may extend from the upper abdomen of a patient to the lower chest, the upper abdomen and the lower chest meeting at the lung sinus and being divided from one another by the diaphragm.

The invention also extends to an apparatus for performing such a method and therefore, viewed from another aspect, the invention provides an apparatus for monitoring the position of a patient's diaphragm comprising: an array of at least two ultrasound transducer elements for placing on the patient in the direction of the longitudinal (z) axis of the patient to extend over the lung sinus, wherein each individual transducer element is arranged to detect an ultrasound beam which is reflected from tissue adjacent thereto and to provide an output signal;
wherein the apparatus is arranged to measure the strength of each output signal to obtain a value for the acoustic impedance of said tissue adjacent to the transducer element, and to determine the position of the diaphragm based upon a comparison of the measured acoustic impedance values

The array preferably comprises low cost disposable transducer elements. The array is preferably fixed to the patient's skin in a lower intercostal space using an adhesive. In accordance with standard practice, a coupling medium (ultrasound gel) should preferably be applied to the patient's skin under the transducer elements.

In preferred forms of the invention the transducer array may indicate its own proper placement by giving feedback to the user. Thus, in one preferred method, the array of ultrasound transducer elements is placed over the patient's lower chest and/or upper abdomen and is moved to a desired location over the lung sinus using feedback from the ultrasound transducer elements.

In one preferred embodiment, the array of transducer elements is made long enough to extend across the full extent of movement of a patient's diaphragm, i.e. to cover a patient's full breathing range. In an alternative embodiment however, the array of transducer elements may be shorter than the full breathing range of a patient, so as for example to cover 50% of a patient's full breathing range. In one possible embodiment, which is not claimed, a single transducer element could be used rather than an array but this would require cooperation from a patient to hold their breath while the diaphragm was located and the transducer element positioned thereon. Further, it is believed that the use of a single transducer element would not be as accurate as it would be very difficult to position the transducer element directly on the edge of the diaphragm. Therefore, at least two transducer elements are used. This has the advantage that a transducer element can be placed on either side of the diaphragm to give a user the certainty that the diaphragm is located between the two elements when the difference in reading obtained by the two elements is sufficiently high.

Although the transducer array of the present invention need only be a one dimensional array in the direction of the longitudinal (z) axis of the patient, a two dimensional array or a plurality of two dimensional arrays could be used if required to more accurately measure the exact two or three dimensional shape and location of an aerated cavity inside a patient.

In operation, the transducer array will start monitoring the movement of the lung sinus; each transducer element will detect a change in impedance as the patient breathes. The output from the transducer array can be registered for later use or constantly fed to a monitor or device.

In a simple case the outputs from the transducer elements may be regarded as essentially digital-in simple terms the output from each may be regarded as either indicating air (above the diaphragm) or indicating tissue (on or below the diaphragm). These outputs may then be fed to a processor that determines the position of the "step" from one to the other. This will correspond to diaphragm position. An output may then be made on a display, for example as a numerical value on an arbitrary scale.

In a more sophisticated embodiment the measured acoustic impedance from each transducer element is used as an input to the processor. Impedance may then be processed as a function that varies with the z-direction. The impedance values may, for example, be plotted on a visual display and a curve fitted to them. The diaphragm position will correspond to the point of greatest gradient. This can either be determined visually by an operator or calculated and presented as a position-value.

Using this apparatus it is possible to monitor the position of the lung sinus and thereby the diaphragm with great accuracy in real time. Procedures such as lung biopsies may therefore be performed with greater ease.

Moreover, in the context of radiotherapy, the apparatus may be coupled to a source of radiation and be arranged to trigger that source to emit radiation when the diaphragm is at a specific position. In this way, the radiation need only be directed at the patient when it is directly focused on its target. In one technique that can be used with embodiments of the invention, the direction of the radiation beam may be controlled to follow the movement of the target based on the determined position of the diaphragm.

In one such technique, the location of the tumor is measured at several different levels of inspiration and the path of the tumor throughout a normal breathing cycle is calculated as a function of diaphragm position. The radiation source is mounted on a tracking device that is controlled by the control unit to direct the radiation towards the calculated position of the tumor based on the current measurement of diaphragm position.

Furthermore, the inventors have recognized that when monitoring the respiratory status of seriously ill patients, e.g. in the intensive care unit (ICU) it would be useful to measure the position of the diaphragm by means of the present invention. This would give important information about the quality of respiration that is not available with the techniques currently used in the ICU.

A preferred embodiment of the present invention will now be described, by way of example only, with reference to the accompanying drawings in which:-
Fig. 1 shows a schematic representation of an embodiment of the invention using an array of ultrasound transducer elements to detect the location of a patient's diaphragm;

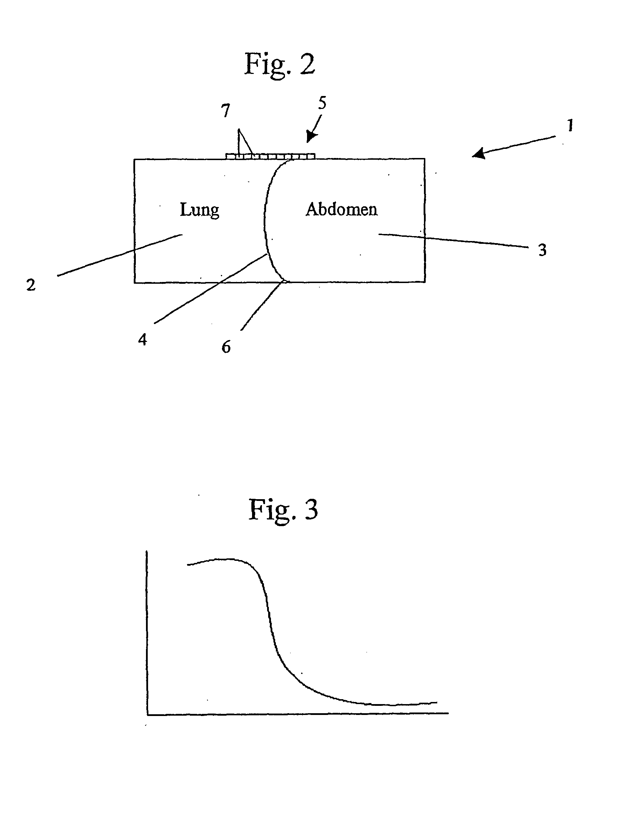

Fig. 2 shows the diaphragm in a contracted state (after inspiration) ;
Fig. 3 illustrates output from the embodiment in the form of a graph illustrating acoustic impedance plotted against the z-direction with the diaphragm in a contracted state;

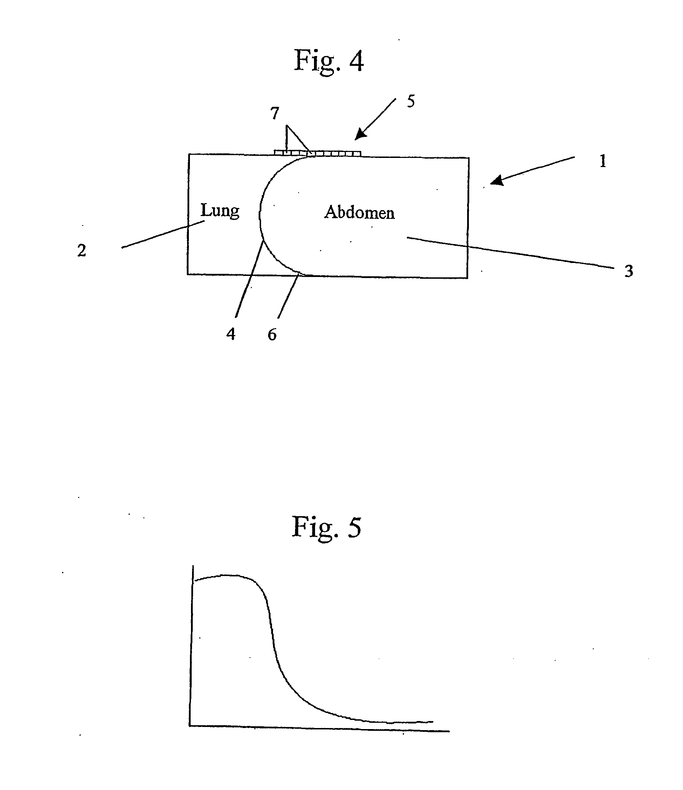

Fig. 4 corresponds to Fig. 2 but shows the diaphragm in a relaxed state (after expiration);
Fig. 5 corresponds to Fig. 3 but shows an impedance signal corresponding to the diaphragm state shown in Fig. 4;
Fig. 6 shows a system for implementing the embodiment of Figure 1 illustrating how the invention may be utilised.

For the sake of clarity, the present invention will now be described with reference to a CT guided puncture operation. However, it will be understood that, as discussed above, embodiments of the invention can also be used in the fields of radiation therapy and other aspects of respiration monitoring.

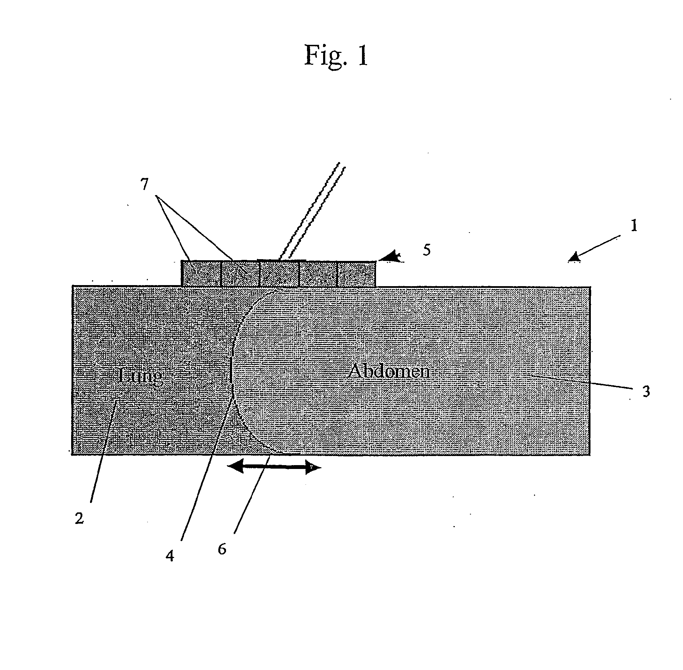

Fig. 1 shows a respiration monitoring apparatus in accordance with the present invention. A patient 1 having a lung 2 and an abdomen 3 being separated therefrom by a diaphragm 4 is fitted with an ultrasound transducer array 5 positioned approximately centrally over the lung sinus 6 prior to being given a CT or MRI scan. The transducer array 5 consists of a plurality of individual transducer elements 7 that span at least a part of the region of diaphragm movement i.e. from a relaxed state to a contracted state. In one embodiment, the transducer array is adapted to span the entire region of diaphragm movement. In alternative embodiments however, it can be adapted to span only 75%, 50% or 25% of the region of diaphragm movement respectively. In further possible embodiments, the array can be adapted to span from 1% to 15% or from 1% to 20% or from 1% to 25% of the region of diaphragm movement respectively.

Each individual transducer element 7 emits an ultrasound pulse and then detects its echo in the known manner. Because air has a much higher acoustic impedance than tissue, the reflection of the ultrasound beam is much more pronounced when the lung is insonated. Thus, the strength of signal received will be much higher when the pulse is reflected from an aerated space than when the pulse is reflected by tissue.

By measuring the strength of the receiving signal, i.e. the degree to which the transducers are receiving from air and from tissue, it is possible to determine to a high degree of accuracy the position of the patient's diaphragm.

In order to avoid interference between adjacent transducer elements, their pulses are phased so as not to occur simultaneously. However, because the duration of each pulse is very short, output that is effectively in real-time is produced.

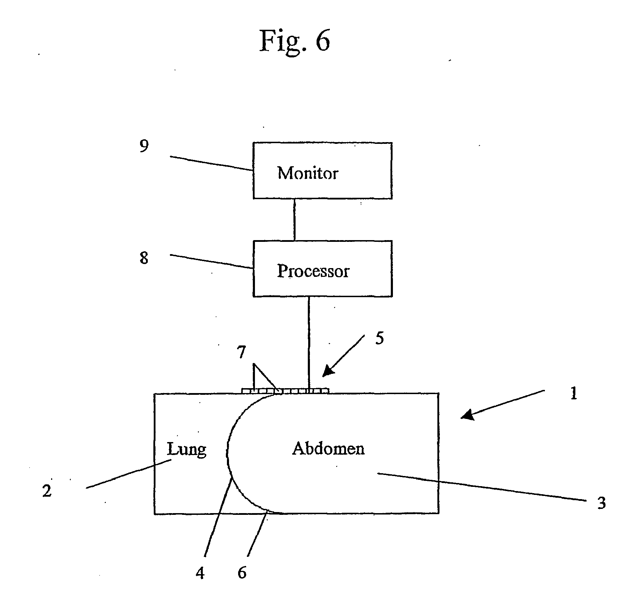

As shown in Figure 6, the measured acoustic impedance from each transducer element is used as an input to a processor 8..Impedance may then be processed as a function that varies in the z-direction. The impedance values are then plotted on a visual display 9 and a curve fitted to them. The diaphragm position will correspond to the point of greatest gradient. This is calculated by the processor 8, highlighted on the display 9 and presented as a numerical position-value, also on the display 9..

After the patient 1 has been fitted with the transducer array 5, a CT or an MRI scan is performed on the patient to determine the precise location of the target (e.g. a lesion to be punctured). During the scan, the patient is required to hold his breath so that a clear image is produced with the lungs in one position. While the scan is being performed and while the patient is holding his or her breath, the exact position of the diaphragm is presented on the display and the position-value is noted.

The image from the scan is used to calculate the depth and angle at which a needle must be inserted for the lesion to be punctured. When the operator is ready to perform the puncture, the patient is asked to inhale until the display indicates that the diaphragm is in the same position as it was when the scan was performed. If the patient inhales too much and the transducer array indicates that the level of inspiration is greater than that held during the scan, the operator can instruct the patient to exhale a little. If necessary, the patient can relax and inhale again until the operator is happy with the position of the diaphragm.

In this way, the operator can be sure that the lesion is at the same position within the patient as it is shown in the CT or MR image while he or she performs the puncture. In the case of CT, the location of the needle may, however, still be checked by means of a further scan.

As described above, the apparatus of the present invention can also be used to improve radiotherapy treatments by reducing the area that needs to be irradiated. The basic procedure described above is employed, however, the embodiment is modified to provide a control output from the processor for controlling a source of radiation.

After the location of the tumor within the patient has been determined from the scan image, a radiation source is aimed at that location. This is connected to the control output such that the radiation source only emits when triggered to do so by the output signal from the processor.

The patient is allowed to breathe continuously throughout the radiation treatment. Meanwhile, the processor uses the outputs from the transducer array to continuously monitor the position of the diaphragm. When its position corresponds to the position that was determined during the scan, the processor sends a signal to trigger the radiation source to irradiate the target area of the patient. Thus, the area of the patient that needs to be irradiated can be significantly reduced because the location of the target can be determined to a much greater accuracy.

## Claims

1. A method of determining the degree of lung inspiration in a patient (1) comprising the step of non-invasively detecting the position of the patient's diaphragm (4) by means of an array of at least two ultrasound transducer elements (7) on the patient extending in the direction of the longitudinal (z) axis of the patient over the lung sinus (6), wherein: each individual transducer element detects an ultrasound beam which is reflected from tissue adjacent thereto and provides an output signal;
and comprising the step of measuring the strength of each output signal to obtain a value for the acoustic impedance of said tissue adjacent to the transducer element; and determining the position of the diaphragm based upon a comparison of the measured acoustic impedance values from each transducer.

2. A method as claimed in claim 1, wherein the diaphragm position is used as a reference point to define the degree of lung inspiration when an image of the patient (1) is generated.

3. A method as claimed in claim 2, wherein the diaphragm position is first determined whilst the patient (1) holds his breath and images are generated simultaneously therewith the degree of lung inspiration subsequently being reproduced by the patient inhaling or inhaling and exhaling until the previously determined diaphragm position is achieved.

4. A method as claimed in any preceding claim, wherein the array of ultrasound transducer elements (7) is placed on the patient's lower chest and/or upper abdomen and is moved into a desired position over the lung sinus (6) using feedback from the ultrasound transducer elements.

5. An apparatus for monitoring the position of a patient's diaphragm (4) comprising: an array of at least two ultrasound transducer elements (7) for placing on the patient (1) in the direction of the longitudinal (z) axis of the patient to extend over the lung sinus (6), wherein each individual transducer element is arranged to detect an ultrasound beam which is reflected from tissue adjacent thereto and to provide an output signal;
wherein the apparatus is arranged to measure the strength of each output signal to obtain a value for the acoustic impedance of said tissue adjacent to the transducer element, and to determine the position of the diaphragm based upon a comparison of the measured acoustic impedance values.

6. An apparatus as claimed in claim 5, wherein the array of transducer elements (7) is a one-dimensional array.

7. An apparatus as claimed in any of claims 5 or 6, wherein the measured acoustic impedance from each transducer element (7) is used as an input to a processor (8) and the acoustic impedance measurements are processed to provide a function that varies with the movement of the diaphragm (4) in the z-direction.

8. A radiotherapy apparatus comprising a radiation source, a control unit, and an apparatus for monitoring diaphragm position as claimed in claim 5, 6 or 7, wherein the source is mounted on a tracking device and the control unit is arranged to control the source to direct the radiation towards the calculated position of a tumor based on the current measurement of diaphragm position.

9. A method of monitoring respiration by monitoring the movement of a patient's diaphragm using the method or apparatus of any of claims 1 to 7.

## Patentansprüche

1. Verfahren zum Bestimmen eines Grades einer Lungeneinatmung bei einem Patienten (1), das den Schritt umfasst, nicht-invasiv die Position des Zwerchfells (4) des Patienten mittels einer Anordnung von mindestens zwei Ultraschallwandlerelementen (7) auf dem Patienten zu bestimmen, die sich in der Richtung der Längsachse (z) des Patienten über den Lungensinus (6) erstrecken, wobei bei dem Verfahren: jedes individuelle Wandlerelement einen Ultraschallstrahl detektiert, der von Gewebe, das an dem jeweiligen Wandlerelement angrenzt, reflektiert wird, und ein Ausgabesignal bereitstellt;
und das den Schritt umfasst, die Stärke jedes der Ausgabesignale zu messen, um einen Wert für die akustische Impedanz des an dem Wandlerelement anliegenden Gewebes zu ermitteln; und die Position des Zwerchfells auf Grundlage eines Vergleichs der von jedem der Wandler gemessenen akustischen Impedanzwerte zu bestimmen.

2. Verfahren nach Anspruch 1, bei dem die Zwerchfellposition als ein Referenzpunkt verwendet wird, um den Grad der Lungeneinatmung zu definieren, während ein Bild des Patienten (1) erzeugt wird.

3. Verfahren nach Anspruch 2, bei dem die Zwerchfellposition zunächst bestimmt wird, während der Patient (1) seinen Atem anhält und dabei gleichzeitig Bilder erzeugt werden, wobei der Grad der Lungeneinatmung anschließend dadurch reproduziert wird, dass der Patient einatmet oder einatmet und ausatmet, bis die zuvor bestimmte Zwerchfellposition erreicht ist.

4. Verfahren nach einem der vorhergehenden Ansprüche, bei dem die Anordnung der Ultraschallwandlerelemente (7) auf dem unteren Brustbereich und/oder dem oberen Abdomen platziert wird und unter Verwenden einer Rückmeldung von den Ultraschallwandlerelementen in eine gewünschte Stellung über dem Lungensinus (6) bewegt wird.

5. Vorrichtung zum Bestimmen der Position des Zwerchfells eines Patienten (4), die umfasst: Eine Anordnung von mindestens zwei Ultraschallwandlerelementen (7) zum Platzieren auf dem Patienten (1) in der Richtung der Längsachse (z) des Patienten, so dass sie sich über den Lungensinus (6) erstrecken, wobei jedes individuelle Wandlerelement derart angeordnet ist, einen Ultraschallstrahl zu detektieren, der von Gewebe, das an dem jeweiligen Wandlerelement angrenzt, reflektiert wird, und ein Ausgabesignal bereitstellt;
wobei die Vorrichtung dafür eingerichtet ist, die Stärke jedes der Ausgabesignale zu messen, um einen Wert für die akustische Impedanz des an dem jeweiligen Wandlerelement anliegenden Gewebes zu ermitteln, und dafür, die Position des Zwerchfells auf Grundlage eines Vergleichs der gemessenen akustischen Impedanzwerte zu bestimmen.

6. Vorrichtung nach Anspruch 5, bei der die Anordnung von Wandlerelementen (7) eine eindimensionale Anordnung ist.

7. Vorrichtung nach einem der Ansprüche 5 oder 6, bei der die gemessene akustische Impedanz eines jeden Wandlerelements (7) als Eingabe für einen Prozessor (8) verwendet wird und die akustischen Impedanzmessungen verarbeitet werden, um eine Funktion bereitzustellen, die mit der Bewegung des Zwerchfells (4) in der z-Richtung variiert.

8. Strahlentherapievorrichtung, die eine Strahlungsquelle, eine Steuereinheit und eine Vorrichtung zum Beobachten einer Zwerchfellposition nach den Ansprüchen 5, 6 oder 7 umfasst, wobei die Quelle an einer Verfolgungseinrichtung angebracht ist und die Steuereinheit dafür eingerichtet ist, die Quelle derart zu steuern, die Strahlung auf Grundlage einer gegenwärtigen Messung der Zwerchfellposition in Richtung einer berechneten Position eines Tumors zu richten.

9. Verfahren zum Beobachten von Atmung durch Beobachten der Bewegung des Zwerchfells eines Patienten, bei dem ein Verfahren oder eine Vorrichtung nach einem der Ansprüche 1 bis 7 verwendet wird.

## Revendications

1. Procédé de détermination du degré d'inspiration pulmonaire chez un patient (1), comprenant l'étape de détection en mode non invasif de la position du diaphragme (4) du patient au moyen d'un réseau d'au moins deux éléments transducteurs ultrasonores (7) sur le patient s'étendant dans la direction de l'axe longitudinal (z) du patient sur le sinus pulmonaire (6), dans lequel chaque élément transducteur individuel détecte un faisceau ultrasonore qui est réfléchi par le tissu qui lui est adjacent et délivre un signal de sortie ;
et comprenant l'étape de mesure de l'intensité de chaque signal de sortie pour obtenir une valeur pour l'impédance acoustique dudit tissu adjacent à l'élément transducteur ; et de détermination de la position du diaphragme sur la base d'une comparaison des valeurs d'impédance acoustique mesurées issues de chaque transducteur.

2. Procédé selon la revendication 1, dans lequel la position du diaphragme est utilisée comme point de référence pour définir le degré d'inspiration pulmonaire lorsqu'une image du patient (1) est générée.

3. Procédé selon la revendication 2, dans lequel la position du diaphragme est tout d'abord déterminée tandis que le patient (1) retient sa respiration et que des images sont générées simultanément avec cette situation, le degré d'inspiration pulmonaire étant ensuite reproduit par le patient inhalant ou inhalant et exhalant jusqu'à ce que la position du diaphragme précédemment déterminée soit obtenue.

4. Procédé selon l'une quelconque des revendications précédentes, dans lequel le réseau d'éléments transducteurs ultrasonores (7) est placé sur la poitrine inférieure et/ou l'abdomen supérieur du patient et est déplacé dans une position souhaitée sur le sinus pulmonaire (6) en utilisant une rétroaction des éléments transducteurs ultrasonores.

5. Appareil de surveillance de la position du diaphragme (4) d'un patient, comprenant un réseau d'au moins deux éléments transducteurs ultrasonores (7) à placer sur le patient (1) dans la direction de l'axe longitudinal (z) du patient pour s'étendre sur le sinus pulmonaire (6), dans lequel chaque élément transducteur individuel est aménagé pour détecter un faisceau ultrasonore qui est réfléchi par un tissu qui lui est adjacent et pour délivrer un signal de sortie ;
dans lequel l'appareil est aménagé pour mesurer l'intensité de chaque signal de sortie pour obtenir une valeur pour l'impédance acoustique dudit tissu adjacent à l'élément transducteur et pour déterminer la position du diaphragme sur la base d'une comparaison des valeurs d'impédance acoustique mesurées.

6. Appareil selon la revendication 5, dans lequel le réseau d'éléments transducteurs (7) est un réseau unidimensionnel.

7. Appareil selon l'une quelconque des revendications 5 ou 6, dans lequel l'impédance acoustique mesurée issue de chaque élément transducteur (7) est utilisée comme entrée dans un processeur (8) et les mesures d'impédance acoustique sont traitées pour fournir une fonction qui varie avec le mouvement du diaphragme (4) dans la direction z.

8. Appareil de radiothérapie comprenant une source de rayonnement, une unité de commande et un appareil de surveillance de la position du diaphragme selon les revendications 5, 6 ou 7, dans lequel la source est montée sur un dispositif de suivi et l'unité de commande est aménagée de manière à commander la source pour diriger le rayonnement vers la position calculée d'une tumeur sur la base de la mesure courante de la position du diaphragme.

9. Procédé de surveillance respiratoire par surveillance du mouvement du diaphragme d'un patient en utilisant le procédé ou l'appareil selon l'une quelconque des revendications 1 à 7.
